# DEMANDE DE BREVET EUROPEEN

(11) **EP 1 075 842 A1**
(43) Date de publication de la demande: **14.02.2001**
(21) Numéro de dépôt: 99810717.1
(22) Date de dépôt: 09.08.1999
(51) Int. Cl.: A61L 9/12

(54) **Procédé et dispositif pour désodoriser et désinfecter l'air**

(71) Demandeur: Challand, René, 1206 Genève (CH); Trachsel, Alain, 1196 Gland (CH); Trachsel, Josiane, 1196 Gland (CH)
(72) Inventeur: Challand, René, 1206 Genève (CH); Trachsel, Alain, 1196 Gland (CH); Trachsel, Josiane, 1196 Gland (CH)
(74) Mandataire: Moinas, Michel

(57) **Abrégé**

Dispositif pour libérer une substance, en particulier odorante ou désinfectante, dans un volume d'air circulant dans un conduit (1), caractérisé en ce qu'il comprend une canne d'injection (3) montée à l'intérieur du conduit, une bonbonne extérieure (5) contenant la substance et un gaz propulseur sous pression, et une vanne (7) disposée sur un raccord de communication (9) entre la bonbonne et la canne d'injection.

Le gaz propulseur se détend à l'ouverture de la vanne (7) pour transporter une quantité de la substance de la bonbonne (5) dans le conduit à travers la canne d'injection (3).

Le dispositif comprend en outre un capteur de pression (11) déterminant la pression dans la bonbonne et un capteur de débit (13) ou de température déterminant le débit ou la température de l'air circulant dans le conduit. De préférence, la vanne (7) est une électrovanne commandée par un microcontrôleur (17).

Le microcontrôleur contrôle automatiquement l'ouverture de l'électrovanne en fonction de la pression du gaz propulseur dans la bonbonne et du débit d'air circulant dans le conduit.

## Description

L'invention se rapporte à un procédé et à un dispositif pour libérer une substance, en particulier odorante ou désinfectante, dans un volume d'air.

Il existe à l'heure actuelle de nombreux dispositifs de libération de substance odorante qui demandent d'être disposés dans le volume d'air même où l'on libère la substance. Dans ces dispositifs, à l'exemple de bâtons d'encens ou d'appareils de chauffage d'essences naturelles, le transport de la substance dans le volume d'air s'effectue principalement par diffusion et par convection naturelle. Ces modes de transport sont relativement lents, et ne permettent pas de disperser de façon homogène la substance dans le volume d'air. De plus, le réglage de la quantité de substance émise par le dispositif est sommaire et consiste simplement en la mise en service ou l'arrêt du dispositif.

Pendant l'utilisation, on aboutit fréquemment à une concentration excessive ou insuffisante de la substance, selon que l'espace est trop réduit ou trop grand par rapport à la quantité émise par le dispositif. Il n'est pas inutile de rappeler que la quantité de parfum créant une sensation agréable pour l'être humain est habituellement de l'ordre de quelques parties par millions (ppm). Il en va de même de substances désinfectantes, pour lesquelles la quantité libérée ne doit pas être supérieure à un seuil de toxicité.

Pour augmenter l'homogénéité de la dispersion et contrôler plus facilement la quantité de substance émise, on préfère faire circuler un volume d'air dans un conduit et libérer la substance odorante ou désinfectante à l'intérieur du conduit. La circulation de l'air dans le conduit crée une convection forcée qui permet une dispersion plus homogène de la substance dans le volume d'air à traiter.

La substance odorante ou désinfectante est en général solubilisée dans un solvant avec lequel elle forme une composition organique liquide. La composition organique peut aussi être constituée de la substance odorante ou désinfectante à l'état pur. Le liquide est contenu dans une cartouche. Pour disperser la substance dans le volume d'air, on installe la cartouche à proximité du conduit et on force le liquide à l'aide du compresseur à s'éjecter sous forme de fines gouttelettes hors de la cartouche dans un filtre faisant office de diffuseur disposé dans le conduit, la substance odorante étant entraînée par l'air à traiter circulant dans le conduit.

Dans ce type de procédés connus, on relève que le contrôle de la quantité de substance libérée dans le volume d'air présente une inertie importante due au compresseur : il existe un retard entre l'ordre envoyé vers le compresseur et un changement effectif de la quantité de substance libérée dans le volume d'air. Une libération à la demande ou par intermittence pour permettre un dosage fin de la quantité de substance émise n'est pas aisément réalisée avec le compresseur dédié en premier lieu à un régime permanent.

L'un des buts de l'invention est de traiter un volume d'air en écoulement dans un conduit en dispersant une substance odorante ou désinfectante de façon à contrôler facilement la quantité de substance émise et permettre ainsi un dosage fin de la substance dans le volume d'air.

A cet effet, l'invention a pour objet un procédé pour disperser une substance, en particulier odorante ou désinfectante, dans un volume d'air, dans lequel procédé on met en écoulement un volume d'air dans un conduit et on libère une substance odorante ou désinfectante à l'intérieur du conduit, caractérisé en ce que l'on dispose à l'extérieur du conduit une bonbonne contenant la substance à disperser et un gaz propulseur comprimé, et l'on détend le gaz propulseur pour injecter la substance à l'intérieur du conduit.

Le gaz propulseur contenu dans la bonbonne sous pression se détend avec un temps de réponse négligeable. De cette façon, le gaz propulseur entraîne immédiatement la substance odorante ou désinfectante vers la région d'injection du conduit. L'injection rapide permet un dosage fin de la quantité de la substance libérée dans le volume d'air en écoulement dans le conduit. Si l'on doit changer de substance à libérer ou recharger le procédé, l'on procède simplement à un remplacement de la bonbonne sans intervenir dans le conduit.

Avantageusement, selon le procédé de l'invention, on contrôle l'injection de la substance en fonction d'une indication de la pression du gaz propulseur dans l'enceinte et d'une indication du débit d'air en écoulement dans le conduit. On utilise les indications de pression du gaz propulseur et de débit d'air en écoulement pour obtenir une concentration de la substance dispersée dans le volume d'air la plus linéaire possible.

D'une façon également avantageuse, l'on coupe l'injection du gaz propulseur lorsque la pression de l'air dans le conduit passe en dessous d'un seuil bas de pression. Lorsque la pression dans le conduit chute et franchit le seuil bas, l'injection est coupée par sécurité pour éviter une accumulation du gaz propulseur dans le conduit.

L'invention s'étend à un dispositif pour libérer une substance, en particulier odorante ou désinfectante, dans un volume d'air en écoulement dans un conduit, caractérisé en ce qu'il comprend une canne d'injection montée à l'intérieur du conduit, une bonbonne extérieure contenant la substance à disperser et un gaz propulseur sous pression, et une vanne montée sur un raccord de communication entre la bonbonne et la canne d'injection, le gaz propulseur se détendant à l'ouverture de la vanne pour transporter une quantité de la substance à disperser de la bonbonne dans le conduit à travers la canne d'injection.

Avantageusement, la vanne est une électrovanne commandée par un moyen informatique comme un microcontrôleur ou un microprocesseur. Cet agencement permet une gestion souple et un contrôle automatique de la quantité de substance dispersée. Il est prévu de programmer le microcontrôleur pour piloter l'électrovanne à distance à l'aide d'un micro-ordinateur.

Par comparaison avec un compresseur forçant le liquide à s'éjecter hors de la cartouche dans le conduit, la bonbonne contenant la composition organique liquide et le gaz propulseur apporte une réduction de poids et élimine les moments d'immobilisation inévitables de purge, d'entretien et de réparation du compresseur. De surcroît, la commande de l'électrovanne à l'aide d'un microcontrôleur ou d'un microprocesseur se révèle plus simple que la commande d'un compresseur.

D'autres caractéristiques et avantages de l'invention apparaîtront à la lecture de la description détaillée d'un mode de réalisation illustré par les dessins.

La figure 1 est un diagramme schématique d'un dispositif de l'invention.

La figure 2 montre un schéma de programmation d'un microcontrôleur commandant une électrovanne dans un dispositif de l'invention.

Un procédé pour disperser une substance, en particulier odorante ou désinfectante, dans un volume d'air comprend une première étape de mise en circulation du volume d'air dans un conduit et une deuxième étape de libération de la substance dans le conduit. La circulation du volume d'air assure par exemple la ventilation d'un véhicule, d'un restaurant, d'un cinéma ou encore d'un hôpital. La substance libérée dans le conduit se disperse dans le volume d'air par convection forcée et conduit à une ambiance parfumée ou à un air contenant un principe actif désinfectant.

Selon l'invention, l'on dispose à l'extérieur du conduit une bonbonne contenant la substance à disperser et un gaz propulseur comprimé, et l'on détend le gaz propulseur pour injecter la substance à l'intérieur du conduit.

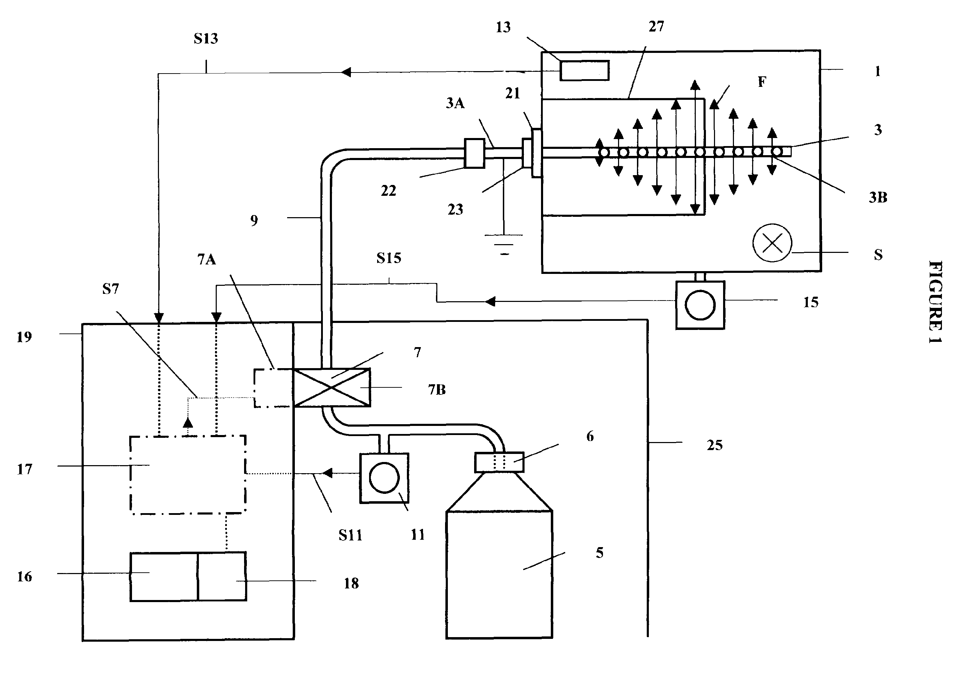

Sur la figure 1, la référence 1 désigne un conduit de ventilation dans lequel circule un volume d'air. La circulation de l'air s'effectue perpendiculairement au plan de la figure 1 comme indiqué par le symbole S en croix disposé dans le conduit. Un dispositif pour libérer une substance odorante ou désinfectante dans le conduit comprend une canne d'injection 3 montée à l'intérieur du conduit 1, une bonbonne 5 disposée à l'extérieur du conduit de ventilation 1 et une vanne 7 disposée sur un raccord de communication 9 entre la bonbonne 5 et la canne d'injection 3. La bonbonne 5 contient la substance solubilisée dans un solvant avec lequel elle forme une composition organique liquide. La composition organique peut aussi être constituée de la substance odorante ou désinfectante à l'état pur.

A l'ouverture de la vanne 7, le gaz propulseur se détend pour injecter la substance à disperser dans le conduit de ventilation 1 à travers le raccord de communication 9 et la canne d'injection 3. L'injection de la substance dans le conduit, symbolisée sur la figure 1 par les flèches F, est effectuée rapidement après l'ouverture de la vanne, du fait de la détente immédiate du gaz propulseur.

Avantageusement, l'on contrôle l'injection de la substance en fonction d'une indication de la pression du gaz propulseur dans la bonbonne et d'une indication du débit d'air circulant dans le conduit. Sur la figure 1, la référence 11 désigne un capteur de pression monté sur le raccord de communication 9 entre la vanne 7 et la bonbonne 5, et la référence 13, un capteur de débit monté sur le conduit de ventilation 1.

Les indications de la pression du gaz propulseur dans la bonbonne 5 et du débit d'air circulant dans le conduit 1 sont utilisées pour obtenir une injection de substance la plus régulière possible. Si le débit d'air diminue dans le conduit, on diminue le temps d'ouverture de la vanne 7. En revanche, si la pression du gaz propulseur diminue dans la bonbonne 5, on augmente le temps d'ouverture de la vanne 7. Cette utilisation des indications données par les capteurs 11 et 13 permet ainsi de maintenir une concentration en substance dispersée dans le volume d'air la plus linéaire possible.

Selon une variante de réalisation, l'indication du débit d'air circulant dans le conduit de ventilation est fixée à une valeur nominale, et un capteur de température, non représenté, est monté dans le conduit pour corriger cette valeur nominale des variations de débit dues à des variations de température. Une correction est notamment utile lorsque le dispositif est installé en zone climatique chaude ou tempérée, sachant qu'une élévation de température diminue le débit d'air massique.

De préférence encore, l'on coupe l'injection du gaz propulseur lorsque la pression de l'air dans le conduit passe en dessous d'un seuil bas de pression. Sur la figure 1, la référence 15 désigne un capteur de pression monté en communication avec le conduit de ventilation 1. Cet agencement permet d'éviter une accumulation du gaz propulseur dans le conduit, ce qui contribue à la sécurité du dispositif, notamment dans le cas où l'on utilise un gaz propulseur inflammable. On enclenche à nouveau l'injection lorsque la pression dans le conduit redevient normale.

Dans le mode de réalisation choisi pour illustrer l'invention, la vanne 7 est une électrovanne commandée par un microcontrôleur ou un microprocesseur 17. Comme illustré par la figure 1, le microcontrôleur 17 et la partie électrique 7A de l'électrovanne 7 sont disposés dans un boîtier 19 qui assure une séparation de ces éléments avec la partie mécanique 7B de l'électrovanne 7, la bonbonne 5 et le raccord de communication 9. Cet agencement augmente la sécurité du dispositif vis-à-vis de risques d'explosion lorsque l'on utilise des gaz inflammables comme gaz propulseur, ou lorsque le dispositif lui-même est disposé dans une ambiance à risque d'explosion. De plus, cet agencement évite toutes perturbations électromagnétiques dans les environs du dispositif, conformément aux normes en vigueur.

Le capteur de pression 11 du gaz propulseur, le capteur de débit 13 ou le capteur de température, et le cas échéant, le capteur de pression 15, du volume d'air circulant dans le conduit, sont reliés au microcontrôleur 17 vers lequel ils envoient des signaux S11, S13 et S15 indicatifs des grandeurs détectées. Le microcontrôleur 17 possède des convertisseurs analogiques-digitaux pour traiter ces signaux selon un algorithme préenregistré dans le microcontrôleur ou chargé dans un micro-ordinateur pilotant le microcontrôleur à distance. L'algorithme est utilisé pour calculer un temps d'ouverture de l'électrovanne.

Cet agencement permet de gérer l'injection de la substance dans le conduit avec une grande souplesse. Outre une injection régulière en fonction du débit ou de la température du volume d'air circulant et de la pression du gaz propulseur, ou encore une coupure de l'injection en cas de franchissement du seuil bas de pression dans le conduit, le microcontrôleur permet de programmer l'injection de la substance en fonction par exemple de plages horaires.

Il est prévu de traiter l'indication de la pression du gaz propulseur dans la bonbonne de façon à ce que le microcontrôleur affiche un signal de remplacement de la bonbonne lorsqu'elle est vide. On peut par exemple stocker dans une mémoire du microcontrôleur la quantité initiale de composition organique liquide contenue dans la bonbonne, ainsi qu'une loi d'extrapolation de la quantité restante en fonction de la diminution de la pression du gaz propulseur.

De préférence, on programme le microcontrôleur par table d'état. Ce type de programmation permet d'obtenir une finesse et une sécurité de haut niveau dans la programmation. La programmation du microcontrôleur utilise des valeurs initiales comme la quantité L de composition organique liquide contenue dans la bonbonne, ou des valeurs nominales, comme le débit d'air Q circulant dans le conduit de ventilation ou la quantité C de substance à disperser dans le volume d'air considéré. Le calcul algorithmique du temps d'ouverture T de l'électrovanne se fonde sur ces valeurs initiales et nominales, sur les valeurs numériques variables résultant de la conversion des signaux envoyés par les capteurs de pression, de débit ou de température, ainsi que sur des lois d'extrapolation ou de correction. Si l'on fixe une nouvelle quantité de substance à disperser dans le volume d'air, le microcontrôleur calcule le nouveau temps d'ouverture de l'électrovanne en fonction de cette nouvelle quantité et des valeurs numériques envoyées par les capteurs.

Comme visible sur la figure 1, les valeurs initiales ou nominales sont saisies par l'intermédiaire d'un clavier à touches 18 sur une face avant du boîtier 19 pour être transmises au microcontrôleur 17. Les différentes valeurs utilisées par l'algorithme sont affichées sur un écran 16 disposé à côté du clavier 18 sur la face avant du boîtier 19.

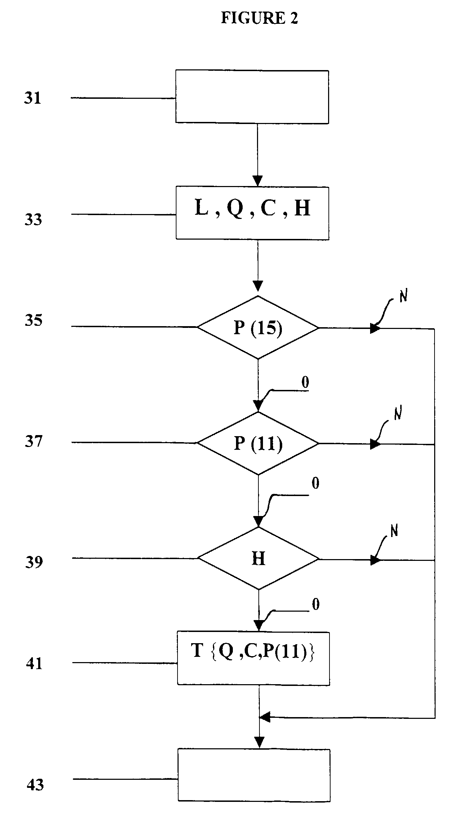

La figure 2 illustre un cycle d'injection exécuté par le microcontrôleur 17 pour commander l'ouverture de l'électrovanne 7. Les références 31 et 43 désignent respectivement le début et la fin du cycle d'injection. La référence 33 désigne l'initialisation du microcontrôleur, au cours de laquelle on fixe la quantité initiale L de composition organique liquide contenue dans la bonbonne 5, le débit d'air Q circulant dans le conduit de ventilation, la quantité C de substance à disperser dans le volume d'air, et la ou les plages horaires H pendant lesquelles la substance doit être dispersée.

La référence 35 désigne un premier test, au cours duquel on contrôle la pression P(15) de l'air à l'aide du capteur de pression 15 monté sur le conduit de ventilation. Si la pression P(15) est inférieure à un seuil bas indiquant que l'air n'est pas en circulation dans le conduit, le microcontrôleur coupe le cycle d'injection pour éviter une accumulation du gaz propulseur dans le conduit non ventilé. La coupure du cycle est symbolisée par la flèche N sur la figure 2. Le microcontrôleur envoie à l'écran 16 du boîtier 19 un message signalant l'absence de circulation d'air dans le conduit. Si au contraire la pression est supérieure au seuil bas, on poursuit le cycle comme indiqué par la flèche O.

La référence 37 désigne un deuxième test, au cours duquel on contrôle la pression P(11) du gaz propulseur dans la bonbonne 5 à l'aide du capteur de pression 11. Si la pression P(11) est inférieure à une valeur limite prédéterminée indiquant que la quantité de composition organique liquide contenue dans la bonbonne est insuffisante, le microcontrôleur coupe le cycle d'injection comme indiqué par la flèche N et envoie à l'écran 16 du boîtier 19 un message signalant la contenance insuffisante de la bonbonne en composition organique liquide. Si au contraire la pression P(11) est supérieure à la pression prédéterminée, on continue le cycle comme indiqué par la flèche O.

La référence 39 désigne un troisième test, au cours duquel on contrôle la plage horaire H pendant laquelle la substance doit être dispersée dans le conduit. Si l'instant du test n'est pas compris dans la plage horaire, le microcontrôleur coupe le cycle et envoie à l'écran un message signalant la position d'attente du dispositif par rapport à la plage horaire enregistrée.

Dans le cas contraire, le microcontrôleur calcule un temps d'ouverture T de l'électrovanne en fonction de la quantité C de substance à disperser dans le volume d'air, du débit d'air Q circulant dans le conduit de ventilation, et de la pression P(11) du gaz propulseur dans la bonbonne. Un ordre d'ouverture S7 est envoyé par le microcontrôleur 17 vers l'électrovanne 7 comme visible sur la figure 1. Le microcontrôleur traite le signal S11 indicatif de la pression du gaz propulseur dans la bonbonne et la valeur nominale Q du débit d'air circulant dans le conduit ou le signal S13 indicatif de ce débit, pour obtenir une quantité C de substance dispersée la plus régulière possible. Comme indiqué précédemment, si le débit d'air diminue dans le conduit, on diminue le temps d'ouverture de la vanne. En revanche, si la pression du gaz propulseur diminue dans la bonbonne, on augmente le temps d'ouverture de la vanne.

La substance à disperser est une composition organique liquide pure ou un principe odorant ou désinfectant contenu en solution dans un solvant, par exemple l'alcool éthylique.

Le gaz propulseur est un mélange du type butane, propane et isobutane pour des applications où le volume d'air n'est pas exposé à des sources de chaleur pouvant provoquer l'inflammation du gaz. Dans le cas de risque d'inflammation, on utilise comme gaz propulseur l'azote, le gaz carbonique, ou l'air lui-même. Il faut noter que les alcanes permettent une plus grande solubilité de la substance organique dans l'air que l'azote ou le gaz carbonique.

La canne d'injection 3 se présente sous la forme d'un tube 3A métallique ou plastique percé de trous d'injection 3B. Le tube 3A est raccordé à une extrémité au conduit de ventilation 1 par l'intermédiaire d'une plaque de fixation 21 et d'un raccord vissé 23 muni d'un joint d'étanchéité. L'extrémité du tube opposée au raccord de communication est bouchée.

Le diamètre des trous d'injection 3B est de préférence plus important dans la région centrale du conduit qu'à proximité de ses parois. Cet agencement favorise la distribution de la substance au centre du conduit et contribue à une plus grande homogénéité de la substance dans le volume d'air. Il faut noter que la canne d'injection 3 est retirée du conduit 1 de façon indépendante de la bonbonne 5 grâce un raccord vissé 22 intermédiaire entre le raccord de communication 9 et le tube 3A. Comme également visible sur la figure 1, la canne d'injection est mise à la terre pour supprimer toute accumulation de charges électriques.

Il est prévu d'utiliser des buses d'injection à la place de la canne d'injection. Il est encore prévu d'utiliser d'autres systèmes de fractionnement en gouttelettes ou de vaporisation de la composition organique, comme par exemple des filtres.

Dans l'exemple d'illustration de l'invention, la bonbonne 5 est protégée par un capot 25 solidaire du boîtier 19. Ce dernier est fixé à une paroi ou monté directement sur le conduit de ventilation. En fonction de la quantité de substance à disperser dans le volume d'air, on utilise des bonbonnes de différentes contenances, typiquement de 100 cm³ à 1000 cm³. Pour un débit d'air circulant dans le conduit ayant une valeur inférieure à 1000 m³ par heure, une seule bonbonne est suffisante pour une utilisation d'une journée à une concentration de quelques ppm de substance dispersée dans le volume d'air. Pour un débit d'air supérieur à 1000 m³ par heure, on prévoit au minimum deux bonbonnes, et un moyen de permutation automatique de l'une à l'autre lorsque toute la composition organique de l'une est injectée, ou pour remplacer une bonbonne par une autre contenant une substance différente, par exemple un parfum différent. Comme indiqué précédemment, l'injection de la substance dans le conduit par la détente du gaz propulseur contenu avec la composition organique dans la bonbonne sous pression permet de réduire au minimum le temps de réponse du dispositif à la dispersion de la nouvelle substance dans le volume d'air circulant dans le conduit.

La vanne 7 est compatible avec la substance à disperser. En particulier, elle est résistante à la corrosion.

Avantageusement, il est prévu de disposer un déflecteur 27 pour perturber l'écoulement laminaire du volume d'air dans le conduit de ventilation 1 et ainsi contribuer à une dispersion plus efficace de la substance.

Le raccord de communication 9 est en acier inoxydable ou en matière plastique, et est prévu pour résister à une surpression. Il est fixé à la bonbonne 5 par un embout 6 également en acier inoxydable ou en matière plastique et permettant, grâce à un clapet interne, de dévisser la bonbonne sans que la substance ne se disperse dans le volume ambiant.

Il faut noter que dans ce mode de réalisation, le dispositif additionne les avantages suivants : faible poids, encombrement réduit, faible consommation électrique et entretien minimum.

Selon un autre mode de réalisation de l'invention, non illustré, le dispositif est embarqué sur un véhicule, et on utilise la batterie du véhicule pour l'alimentation électrique du microcontrôleur et de l'électrovanne. La bonbonne a un volume typique de 100 cm³, et le gaz propulseur est de préférence l'azote ou le gaz carbonique. La canne d'injection est adaptée en dimension au conduit de ventilation du véhicule à l'intérieur duquel elle est montée. La concentration de substance dispersée dans le volume d'air du véhicule est ajustée en fonction de la valeur nominale du débit d'air circulant dans le conduit de ventilation et de la pression dans la bonbonne. Une correction du temps d'ouverture de l'électrovanne en fonction de la température de l'air circulant est aisément réalisée par un capteur de température monté dans le conduit de ventilation.

Il faut noter que le caractère odorant ou désinfectant de la substance à disperser n'est pas en lui-même déterminant pour l'invention. Cette dernière s'applique à toute substance se dispersant dans l'air. N'est pas non plus déterminant le nombre de bonbonnes ou de cannes d'injection.

De même, le conduit de ventilation peut être parcouru par un fluide autre que l'air sans que le procédé et le dispositif décrits précédemment n'en soient modifiés.

Il faut enfin noter que l'invention n'est pas limitée à une mise en mouvement du volume d'air par circulation dans un conduit de ventilation. D'autres écoulements du volume d'air sont possibles. Il est prévu en particulier de mettre en écoulement dans un conduit un volume d'air initialement contenu dans une enceinte sous pression. Le volume d'air est injecté dans le conduit simultanément à l'injection de la substance par la détente du gaz propulseur contenu dans le bonbonne. La durée d'injection de la substance est fixée en fonction de la durée et du débit d'injection du volume d'air dans le conduit. Dans un mode de fonctionnement contrôlé par informatique, le microcontrôleur décrit précédemment est utilisé pour commander à la fois l'ouverture de l'électrovanne de la bonbonne et l'ouverture d'une électrovanne montée sur l'enceinte contenant le volume d'air sous pression.

Ce mode d'écoulement est particulièrement bien adapté pour traiter de petits volumes d'air à la demande. A titre d'exemple, on dispose un conduit débouchant à proximité d'un écran vidéo, et on injecte dans le conduit simultanément un volume d'air et une quantité de substance odorante, dont le parfum est en correspondance avec les images diffusées par l'écran. On crée ainsi autour d'un téléspectateur une ambiance olfactive en association avec l'image vue sur l'écran.

## Revendications

1. Procédé pour disperser une substance, en particulier odorante ou désinfectante, dans un volume d'air, dans lequel procédé on met en écoulement un volume d'air dans un conduit (1) et on libère une substance odorante ou désinfectante à l'intérieur du conduit, caractérisé en ce que l'on dispose à l'extérieur du conduit (1) une bonbonne (5) contenant la substance à disperser et un gaz propulseur comprimé, et l'on détend le gaz propulseur pour injecter la substance à l'intérieur du conduit.

2. Procédé selon la revendication 1, caractérisé en ce que l'on contrôle l'injection de la substance en fonction d'une indication de la pression (P11) du gaz propulseur dans la bonbonne et d'une indication du débit d'air (Q) en écoulement dans le conduit.

3. Procédé selon la revendication 1, caractérisé en ce que l'on coupe l'injection du gaz propulseur lorsque la pression de l'air dans le conduit (P15) passe en dessous d'un seuil bas de pression.

4. Procédé selon les revendications 2 et 3, caractérisé en ce que l'on injecte la substance par l'ouverture d'une électrovanne (7) commandée par un microcontrôleur ou un microprocesseur (17) programmé pour :
a) initialiser (33) une variable représentative de la quantité (L) de substance contenue dans la bonbonne, une variable représentative du débit d'air (Q) en écoulement dans le conduit, et une variable représentative de la quantité (C) de substance à disperser dans le volume d'air,
b) contrôler sous la forme d'un premier test (35) la pression de l'air (P15) dans le conduit,
c) contrôler sous la forme d'un deuxième test (37) la pression (P11) du gaz propulseur dans la bonbonne,
d) ouvrir l'électrovanne (7) pendant un temps d'ouverture (T) calculé en fonction de la pression (P11) du gaz propulseur, du débit d'air (Q) en écoulement dans le conduit, et de la quantité de substance (C) à libérer dans le volume d'air.

5. Procédé selon l'une des revendications précédentes, caractérisé en ce que l'on met le volume d'air en écoulement dans le conduit (1) par une injection à partir d'une enceinte comprimée contenant l'air sous pression, ou par une circulation.

6. Dispositif pour libérer une substance, en particulier odorante ou désinfectante, dans un volume d'air en écoulement dans un conduit (1), caractérisé en ce qu'il comprend une canne d'injection (3) montée à l'intérieur du conduit, une bonbonne (5) extérieure contenant la substance à disperser et un gaz propulseur sous pression, et une vanne (7) montée sur un raccord de communication (9) entre la bonbonne et la canne d'injection, le gaz propulseur se détendant à l'ouverture de la vanne pour transporter une quantité de la substance à disperser de la bonbonne dans le conduit à travers la canne d'injection.

7. Dispositif selon la revendication 6, caractérisé en ce qu'il comprend en outre un capteur de pression (11) déterminant la pression dans la bonbonne et un capteur de débit (13) déterminant le débit d'air en écoulement dans le conduit.

8. Dispositif selon la revendication 6, caractérisé en ce qu'il comprend en outre un capteur de pression (11) déterminant la pression dans la bonbonne et un capteur de température déterminant la température de l'air en écoulement dans le conduit.

9. Dispositif selon la revendication 6, caractérisé en ce qu'il comprend en outre un capteur de pression (15) déterminant la pression dans le conduit.

10. Dispositif selon la revendication 6, caractérisé en ce que la vanne (7) est une électrovanne commandée par un microcontrôleur ou un microprocesseur (17).

11. Dispositif selon la revendication 10, caractérisé en ce que le microcontrôleur (17) est relié à un capteur (11) indicatif de la pression du gaz propulseur dans la bonbonne (5) et à un capteur (13) indicatif du débit d'air en écoulement dans le conduit (1).

12. Dispositif selon la revendication 6, caractérisé en ce que la canne d'injection (3) est munie de trous (3B) dont le diamètre est d'autant plus grand que le trou est disposé dans une partie de la canne plus centrale par rapport au conduit (1).

13. Application du dispositif selon la revendication 10, caractérisée en ce que l'on embarque le dispositif sur un véhicule en installant la canne d'injection (3) dans un conduit de ventilation (1) du véhicule, on injecte la substance dans le conduit par la détente d'un gaz propulseur, et on alimente l'électrovanne (7) et le microcontrôleur ou le microprocesseur (17) à l'aide d'une batterie autonome embarquée sur le véhicule.
